# FASCICULE DE BREVET EUROPEEN

(11) **EP 3 930 768 B1**
(45) Date de publication et mention de la délivrance du brevet: **17.04.2024**
(21) Numéro de dépôt: 20711505.6
(22) Date de dépôt: 26.02.2020
(51) Int. Cl.: A61L 2/10, A61B 1/00, A61B 90/90, G06K 19/00

(54) **PROCÉDÉS DE DÉTECTION D'UN INSTRUMENT MÉDICAL DANS UN KIT DE DÉSINFECTION ET KIT DE DÉSINFECTION METTANT EN OUVRE LES PROCÉDÉS**
VERFAHREN ZUR DETEKTION EINES MEDIZINISCHEN INSTRUMENTS IN EINEM DESINFEKTIONSKIT UND DESINFEKTIONSKIT ZUR DURCHFÜHRUNG DES VERFAHRENS
METHODS FOR DETECTING A MEDICAL INSTRUMENT IN A DISINFECTION KIT AND DISINFECTION KIT IMPLEMENTING THE METHODS

(30) Priorité: 27.02.2019 FR 1902021
(43) Date de publication de la demande: 05.01.2022
(73) Titulaire: Germitec, 94200 Ivry Sur Seine (FR)
(72) Inventeur: NEVEU, Cédric, 92120 Montrouge (FR); LEPINE, Frédéric, 95210 Saint Gratien (FR); DESHAYS, Clément, 75006 Paris (FR)
(74) Mandataire: Lavoix
(86) Numéro de dépôt international: PCT/EP2020/054989
(87) Numéro de publication internationale: WO 2020/173981

(56) Documents cités:
- WO-A1-2009/003231
- WO-A1-2016/001776
- US-A1- 2014 341 777
- US-B2- 8 946 653

## Description

### Domaine technique de l'invention

La présente description concerne généralement des procédés de détection d'un instrument médical dans un système de désinfection, et plus précisément un système de désinfection par rayonnements UV. La présente description concerne également des systèmes de désinfection mettant en oeuvre de tels procédés de détection d'un instrument médical ainsi que des kits de désinfection pour des instruments médicaux.

### Etat de la technique

Une désinfection ou une stérilisation appropriée d'instruments médicaux réutilisables est importante pour empêcher la transmission de microbes pathogènes d'une personne à une autre. Le niveau de stérilisation et de désinfection appliqué aux instruments médicaux dépend de la classification de l'instrument.

On distingue notamment des classes d'instruments critiques qui entrent en contact avec un tissu stérile, par exemple les instruments chirurgicaux, les implants et les sondes à ultrasons utilisés dans des cavités corporelles stériles. Ces instruments doivent être stérilisés ou, à minima, subir une Désinfection de Niveau Haut (DNH) (ou « HLD » pour High-Level Disinfection (HLD) pour les anglo-saxons), avant utilisation. On distingue des classes d'instruments semi-critiques qui entrent typiquement en contact avec les muqueuses ou la peau non intacte. Les instruments semi-critiques donnés à titre d'exemple comprennent des dispositifs tels que les sondes utilisées dans les examens vaginaux, rectaux et urologiques, le matériel de thérapie respiratoire et d'anesthésie et certains endoscopes. Ces dispositifs médicaux doivent être exempts de tout microorganisme, à l'exception d'un faible nombre de spores. Les instruments semi-critiques nécessitent au moins une Désinfection de Niveau Intermédiaire (DNI), ou « ILD » pour Intermediate-Level Disinfection. Les instruments non critiques sont ceux qui entrent en contact avec des membranes non muqueuses d'une peau intacte (par exemple, des brassards de pression sanguine et des stéthoscopes).

Certaines méthodes courantes pour réaliser la stérilisation ou la Désinfection de Niveau Haut d'instruments critiques ou semi-critiques incluent les traitements utilisant des désinfectants vapeur et / ou des désinfectants chimiques (voir par exemple le système de désinfection par désinfectants chimiques trophon^{®} de la société Nanosonics^{®}).

Sont également connus des systèmes capables de réaliser une Désinfection de Niveau Haut d'instruments médicaux réutilisables au moyen de rayonnements ultraviolet (UV). Par rapport aux traitements utilisant des désinfectants vapeur et / ou chimiques tels que décrits précédemment, ces techniques permettent notamment une Désinfection de Niveau Haut rapide, à basse température, sans produits potentiellement toxiques pour le personnel les manipulant et dans les conditions cliniques d'utilisation.

Par exemple, la demande de brevet publiée US2014341777 au nom de la déposante décrit un système de désinfection utilisant une chambre de désinfection avec une source de rayonnement UV.

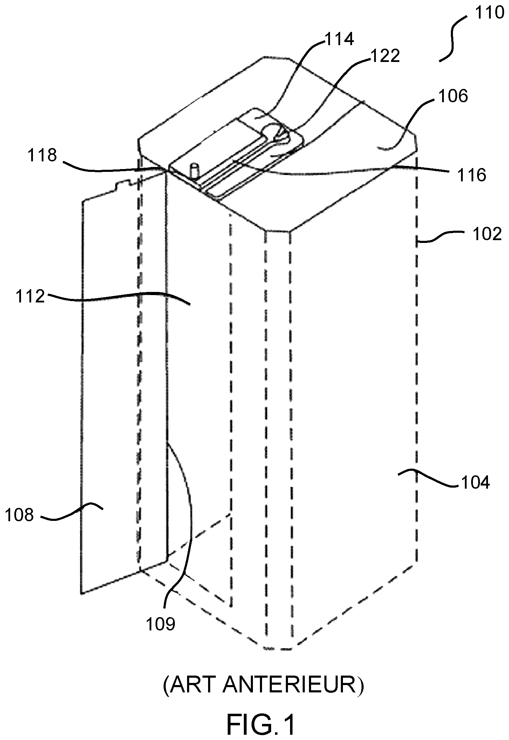

La FIG. 1 décrit un une chambre de désinfection 110 d'un système de désinfection telle que divulgué dans la demande US2014341777 précitée. La chambre de désinfection comprend généralement une enceinte 102 avec une pluralité de parois latérales 104, un plafond 106, une porte 108 agencée par exemple sur l'une des parois latérales 104 pour accéder à un volume intérieur 112 de la chambre de désinfection au moyen d'une ouverture 109 formée dans une des parois. Dans l'exemple de la FIG. 1, un assemblage de suspension 114 est prévu pour suspendre un instrument médical à désinfecter, lors d'un cycle de désinfection, dans une région centrale du volume intérieur de la chambre 110 dans laquelle des radiations UV de forte intensité sont générées. Ainsi par exemple, lorsque l'instrument médical à désinfecter (non représenté sur la FIG. 1) comprend une tête (ou partie active) et un câble de raccordement, l'assemblage de suspension peut comprendre une fente 116 débouchant dans l'ouverture 109 et à une extrémité de laquelle se trouve une ouverture 122 dans laquelle peut passer le câble d'un instrument médical. Par exemple, l'ouverture 122 peut coopérer avec une bague fixée sur le câble de raccordement, à une position déterminée pour que la tête se trouve dans la chambre de désinfection dans une position qui permette une désinfection optimale.

Dans d'autres exemples de réalisation (non représentés), l'assemblage de suspension peut comprendre une pince configurée pour pincer le câble de raccordement à une position déterminée.

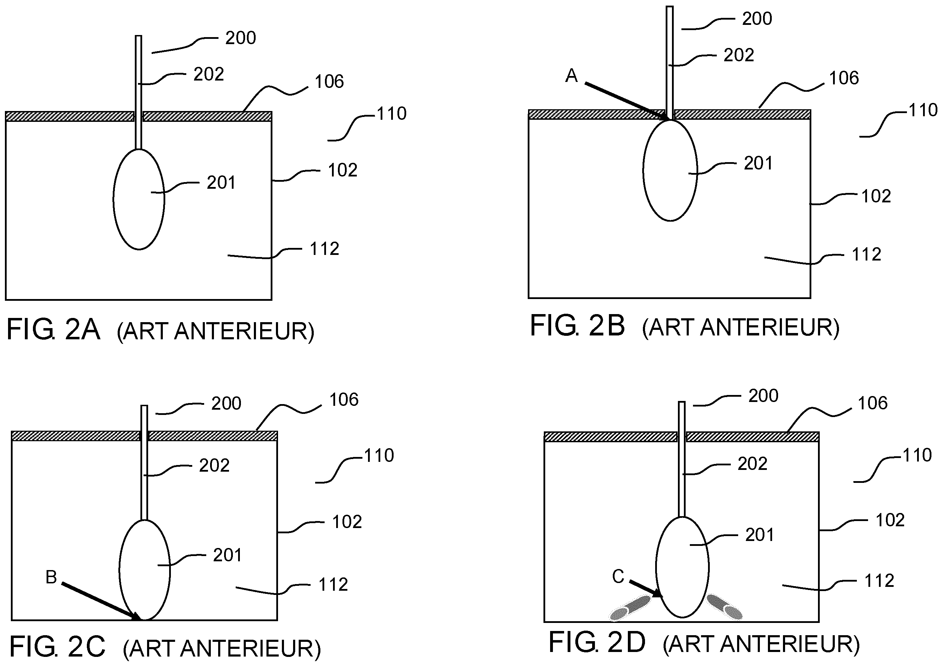

Cependant, quel que soit le mode de positionnement de l'instrument médical dans l'enceinte de désinfection, la déposante a mis en évidence certaines dérives lors de l'utilisation des systèmes de désinfection résultant d'un mauvais positionnement de l'instrument médical. Comme illustré sur les FIGS 2A à 2D, le mauvais positionnement peut résulter par exemple d'un glissement axial du câble de raccordement dans l'assemblage de suspension, ce glissement pouvant entraîner un déplacement incontrôlé de la position de la tête dans la chambre, ou d'une erreur manuelle dans l'accroche de l'instrument.

Les FIGS 2A à 2D illustrent ainsi par des schémas des situations dans lesquelles un instrument médical 200, formé d'une tête 201 et d'un câble de raccordement 202, se trouve suspendu à différentes positions dans le volume intérieur 112 d'une chambre de désinfection 110 par radiations UV.

Dans l'exemple de la FIG. 2A, l'instrument médical 200 est suspendu de telle sorte que la tête 201 se trouve sensiblement positionné dans une région centrale du volume intérieur de la chambre 110, par exemple une position préalablement déterminée pour une désinfection optimale. La position préalablement déterminée pour une désinfection optimale est par exemple une position permettant de délivrer une dose suffisante d'UV sur le point le moins exposé de la tête de l'instrument pour y atteindre une désinfection de haut-niveau, et par conséquent assurer une désinfection de haut-niveau sur toute la surface de la tête de l'instrument. Dans l'exemple de la FIG. 2B, pour une raison ou pour une autre, le câble de raccordement 202 n'est pas ou n'est plus fixé par le même point dans l'assemblage de suspension de sorte que la tête 201 se trouve à proximité du plafond. La région de la tête 201 indiquée par la flèche A ne peut plus recevoir la quantité optimale attendue de rayonnements UV nécessaire à sa désinfection. Dans l'exemple de la FIG. 2C, la tête 201 se trouve à proximité du fond de la chambre 110. Là encore, la région de la tête 201 indiquée par la flèche B ne peut plus recevoir la quantité optimale attendue de rayonnements UV nécessaire à sa désinfection. Comme illustré sur la FIG. 2D, dans cette même position de l'instrument médical 200 vers le fond de la chambre, certaines régions de la tête 201 indiquées par la flèche C pourraient au contraire recevoir une quantité beaucoup plus importante de rayonnements UV par exemple, du fait de la position des sources UV notamment, risquant d'entraîner une détérioration de la tête 201 de l'instrument.

Ainsi, une position non contrôlée de l'instrument médical pendant un cycle de désinfection peut résulter en une désinfection non maîtrisée, parfois insuffisante de l'instrument médical. Non détectée, cette mauvaise position peut ainsi entraîner une réutilisation d'un instrument médical après désinfection dans des conditions sanitaires non satisfaisantes. Des autres systèmes de désinfection sont connus de WO 2016/001776 A1, US 8 946 653 B2, et WO 2009/003231 A1.

Un objet de la présente description est notamment de remédier aux inconvénients précités au moyen de systèmes de désinfection originaux et de procédés de détection d'un instrument médical dans de tels systèmes de désinfection, lesdits procédés visant à vérifier que l'instrument médical est dans une position prédéterminée lors d'un cycle de désinfection.

### Résumé de l'invention

La présente invention est dirigée vers un kit de désinfection selon la revendication 1 et un procédé selon la revendication 8. La présente description peut inclure des autres aspects qui font pas nécessairement partie de l'invention.

Selon un premier aspect, la présente description concerne un système de désinfection configuré pour mettre en oeuvre un cycle de désinfection d'un instrument médical par rayonnements UV. Le système de désinfection comprend une chambre de désinfection configurée pour recevoir au moins une partie dudit instrument médical, un organe de suspension permettant de suspendre ladite au moins une partie de l'instrument médical dans ladite chambre de désinfection, un dispositif de détection configuré pour détecter, dans une zone de détection prédéterminée du système de désinfection, un marqueur solidaire dudit instrument médical et un contrôleur configuré pour générer un signal d'absence de détection en cas de non détection dudit marqueur dans ladite zone de détection prédéterminée.

Par « marqueur solidaire », on comprend un marqueur fixe qui ne puisse être retiré et/ou déplacé sans détérioration dudit marqueur et/ou de l'instrument médical sur lequel il est apposé. Ainsi, le marqueur peut être un élément externe (tel qu'un étiquette, une puce électronique) apposé de façon solidaire à l'instrument médical, par exemple par collage ou intégration au sein de l'instrument médical, ou un élément de l'instrument médical lui-même.

Un système de désinfection ainsi conçu permet d'alerter sur une anomalie dans le positionnement de l'instrument médical et donc possiblement sur un risque de désinfection non optimale. En effet, le fait que le marqueur soit solidaire de l'instrument médical permet de contrôler de façon certaine sa position dans le système de désinfection indépendamment du type de moyens de désinfection.

Selon un ou plusieurs exemples de réalisation, ladite zone de détection du marqueur est déterminée pour s'assurer que ladite au moins une partie de l'instrument médical reçoit une dose minimale d'UV sur sa partie la moins exposée, par exemple une dose permettant une désinfection de haut niveau. La détection du marqueur permet par conséquent de s'assurer d'une désinfection de haut-niveau sur toute la partie de l'instrument destinée à être reçue dans la chambre de désinfection.

Selon un ou plusieurs exemples de réalisation, le dispositif de détection comprend un dispositif de détection optique. Cependant, d'autres types de dispositif de détection sont possibles. Ainsi, selon un ou plusieurs exemples de réalisation, le dispositif de détection peut comprendre des moyens d'acquisition radiofréquence.

Selon un ou plusieurs exemples de réalisation, le contrôleur est configuré pour générer, à partir du signal d'absence de détection une ou plusieurs actions choisie(s) parmi : émission d'une alerte à destination d'un ou plusieurs utilisateur(s), interdiction de lancement d'un cycle de désinfection, un arrêt (blocage) d'un cycle de désinfection en cours.

Selon un ou plusieurs exemples de réalisation, le dispositif de détection, qu'il soit un dispositif de détection optique ou radiofréquence, peut être configuré en outre pour détecter, dans ladite zone de détection, une position du marqueur par rapport à une position de référence.

Selon un ou plusieurs exemples de réalisation, le contrôleur est configuré pour générer, à partir de ladite position du marqueur dans la zone de détection, une ou plusieurs actions comprenant : émission d'une alerte de défaut de positionnement à destination d'un ou plusieurs utilisateur(s) et/ou reconfiguration d'un cycle de désinfection pour tenir compte de ladite position et/ou arrêt du cycle de désinfection en cours et, optionnellement, passage en mode erreur, et/ou interdiction de lancement d'un cycle de désinfection.

Selon un ou plusieurs exemples de réalisation, le dispositif de détection est un dispositif de détection optique, c'est-à-dire un détecteur sensible à un rayonnement visible ou invisible, tel que, par exemple, un rayonnement infrarouge.

Un dispositif de détection optique d'un système de désinfection selon la présente description peut comprendre par exemple et de façon non limitative un détecteur choisi parmi le groupe comprenant : un détecteur ponctuel, par exemple une photodiode, un détecteur matriciel, par exemple une caméra linéaire ou une caméra bidimensionnelle, par exemple une caméra CCD ou CMOS, par exemple un lecteur code-barres à une ou deux dimensions.

Bien entendu, le dispositif de détection est adapté en fonction du marqueur. Une caméra est par exemple adaptée à un marqueur formé d'un élément structurel de l'instrument médical lui-même et identifié comme marqueur pour l'instrument. Par exemple, un détecteur ponctuel tel qu'une photodiode est adapté à un marquer ponctuel, par exemple sous la forme d'un point rouge apporté sur le câble, tandis qu'un lecteur code-barres est adapté à un marqueur de type code-barres.

Selon un ou plusieurs exemples de réalisation, le dispositif de détection est un dispositif de détection optique et comprend en outre une source d'émission d'un rayonnement lumineux configurée pour éclairer ladite zone de détection prédéterminée. La source d'émission permet la détection même lorsque, pendant un cycle de fonctionnement, la zone de détection se trouve dans une région non éclairée du système de désinfection, par exemple un espace fermé à la lumière du jour.

Selon un ou plusieurs exemples de réalisation, le dispositif de détection est configuré pour détecter un marqueur situé dans ladite partie suspendue dans la chambre de désinfection pendant un cycle de désinfection et/ou dans une partie de l'instrument médical qui reste à l'extérieur de la chambre de désinfection pendant un cycle de désinfection.

Le système de désinfection selon le premier aspect est configuré pour mettre en oeuvre un cycle de désinfection par rayonnements UV. Le système de désinfection peut comprendre alors tout moyen connu de l'état de l'art pour mettre en oeuvre un cycle de désinfection par rayonnements UV, et notamment au moins une source de rayonnements UV.

Selon un deuxième aspect, la présente description concerne un kit de détection pour un instrument médical destiné à être désinfecté dans un système de désinfection selon le premier aspect. Le kit de détection comprend un marqueur configuré pour être apposé solidairement sur ledit instrument médical en vue de sa détection par ledit dispositif de détection.

Un kit de détection selon le deuxième aspect peut être utilisé par un opérateur en amont d'un cycle de désinfection pour « marquer » un instrument médical en vue de sa détection dans le système de désinfection avant et/ou pendant et/ou après un cycle de désinfection.

Le marqueur peut comprendre tout élément de nature à pouvoir être détecté de façon spécifique par le dispositif de détection.

Par exemple, lorsque le dispositif de détection est un dispositif de détection optique, le marqueur peut comprendre tout élément « visible » par le détecteur, optionnellement lorsqu'il est éclairé par une source d'émission du dispositif de détection. Le marqueur peut comprendre selon un ou plusieurs exemples et de façon non limitative un élément choisi parmi le groupe comprenant : un élément coloré, un élément fluorescent, une sérigraphie, un élément réfléchissant, un élément diffractif, un code-barres, ou une combinaison de ces éléments.

Selon un ou plusieurs exemples de réalisation, le marqueur comprend un code d'identification dudit instrument médical. Par exemple, le marqueur comprend un code-barres à une, voire à deux dimensions. Un tel marqueur pourra être détecté par un dispositif de détection optique de type lecteur de code-barres. Une identification de l'instrument pourra comprendre, selon un ou plusieurs exemples de réalisation, un numéro de série et/ou un numéro de modèle. Ainsi, la lecture de l'identification de l'instrument médical pourra être couplée à sa détection dans le système de désinfection.

Selon un ou plusieurs exemples de réalisation, lorsque l'instrument médical comprend une tête et un câble de raccordement de la tête, le marqueur peut être configuré pour être apposé de façon solidaire sur une partie du câble voisine de la tête. Il peut s'agir d'une partie du câble destinée à être suspendue dans la chambre de désinfection lors d'un cycle de désinfection ou une partie du câble destinée à rester en dehors de la chambre de désinfection.

Selon un ou plusieurs exemples de réalisation, le marqueur est un élément configuré pour être apposé de façon solidaire dur ledit instrument médical par collage, moulage ou soudage.

Selon un ou plusieurs exemples de réalisation, le kit de détection comprend en outre une bague de protection dudit marqueur, ladite bague de protection coopérant en outre avec ledit organe de suspension pour la suspension du câble.

Par exemple, lorsque le marqueur comprend un élément permettant une détection optique par le dispositif de détection, ladite bague de protection peut comprendre une fenêtre transparente aux longueurs d'onde utiles pour la détection. Une telle bague de protection permettra de protéger le marqueur, disponible par exemple sous forme d'une étiquette souple, pendant un cycle de désinfection.

La présente invention concerne un kit de désinfection pour un instrument médical comprenant un système de désinfection selon le premier aspect et un kit de détection selon le deuxième aspect.

Selon un quatrième aspect, la présente description concerne un instrument médical marqué, destiné à être désinfecté dans un système de désinfection selon le premier aspect. Ledit instrument médical marqué comprend un marqueur apposé solidairement sur une région dudit instrument médical en vue de sa détection par ledit dispositif de détection. Le marqueur est ainsi formé d'un élément externe à l'instrument et configuré pour la détection de l'instrument médical lorsqu'il est positionné dans le système de désinfection, en vue d'un cycle de désinfection. Selon un ou plusieurs exemples de réalisation, ledit marqueur comprend un code d'identification de l'instrument médical. Un tel instrument médical marqué pourra être fourni par exemple avec le système de désinfection.

De plus, la présente invention concerne un procédé de détection d'un instrument médical en utilisant le kit de désinfection selon l'invention.

Un procédé de détection d'un instrument médical selon la présente description comprend les étapes suivantes :
- la détection dans ladite zone de détection prédéterminée du système de désinfection, au moyen dudit dispositif de détection, dudit marqueur solidaire dudit instrument médical ; et
- en cas de non détection dudit marqueur dans ladite zone de détection prédéterminée du système de désinfection, la génération d'un signal d'absence de détection.

La détection du marqueur peut se faire avant et/ou pendant et/ou à la fin d'un cycle de désinfection.

Selon un ou plusieurs exemples de réalisation, la génération du signal d'absence de détection entraine une ou plusieurs actions comprenant : une interdiction de lancement d'un cycle et/ou une émission d'une alerte d'invalidation du cycle à destination d'un utilisateur et/ou un arrêt du cycle de désinfection en cours.

Selon un ou plusieurs exemples de réalisation, la détection est une détection optique.

Selon un ou plusieurs exemples de réalisation, le marqueur comprend un code d'identification de l'instrument médical et le procédé comprend en outre la lecture du code d'identification au moyen du dispositif de détection.

Selon un ou plusieurs exemples de réalisation, le procédé de détection comprend en outre la détection, dans ladite zone de détection, d'une position du marqueur par rapport à une position de référence.

Selon un ou plusieurs exemples de réalisation, le procédé de détection comprend en outre, à partir de ladite position du marqueur dans la zone de détection, une ou plusieurs actions comprenant : émission d'une alerte de défaut de positionnement à destination d'un ou plusieurs utilisateur(s) et/ou reconfiguration d'un cycle de désinfection pour tenir compte de ladite position, et/ou arrêt du cycle de désinfection en cours et, optionnellement, passage en mode erreur, et/ou interdiction de lancement d'un cycle de désinfection.

Selon un ou plusieurs exemples de réalisation, le procédé comprend en outre une étape préalable de marquage de l'instrument médical, par apposition dudit marqueur de façon solidaire sur ledit instrument médical.

### Brève description des figures

D'autres avantages et caractéristiques de l'invention apparaîtront à la lecture de la description, illustrée par les figures suivantes :
- La FIG. 1 (déjà décrite), un schéma d'une enceinte de désinfection selon l'état de l'art ;
- Les FIGS. 2A à 2D (déjà décrites), des schémas illustrant des positions d'un instrument médical comprenant une tête un câble de raccordement de la tête dans un système de désinfection selon l'état de l'art, dans les cas où la tête est positionnée dans une région centrale et optimisée de la chambre (FIG. 2A), dans le cas où la tête se trouve positionnée dans une région trop haute de la chambre (FIG. 2B) et dans le cas où la tête se trouve positionnée dans une région trop basse de la chambre (FIGS. 2C, 2D) ;
- Les FIGS. 3A, 3B, des schémas illustrant un exemple d'un système de désinfection selon la présente description, avec, dans un premier cas, un instrument médical suspendu et détecté par le dispositif de détection (FIG. 3A) et, dans un deuxième cas, un instrument médical suspendu et non détecté par le dispositif de détection (FIG. 3B) ;

- Les FIGS. 4A, 4B, des schémas illustrant une bague de protection d'un kit de détection d'un instrument médical selon la présente description ;
- Les FIGS. 5A, 5B, des schémas illustrant un exemple de support d'un organe de suspension d'un système de désinfection selon la présente description, destiné à coopérer avec une bague de protection telle qu'illustrée sur les FIGS. 4A, 4B ;
- La FIG. 6, un schéma illustrant un autre exemple de réalisation d'un système de désinfection selon la présente description ;
- Les FIGS. 7A, 7B, 7C, 7D, des schémas illustrant des exemples de mise en oeuvre de procédés de détection d'un instrument médical dans un système de désinfection, selon la présente description.

### Description détaillée

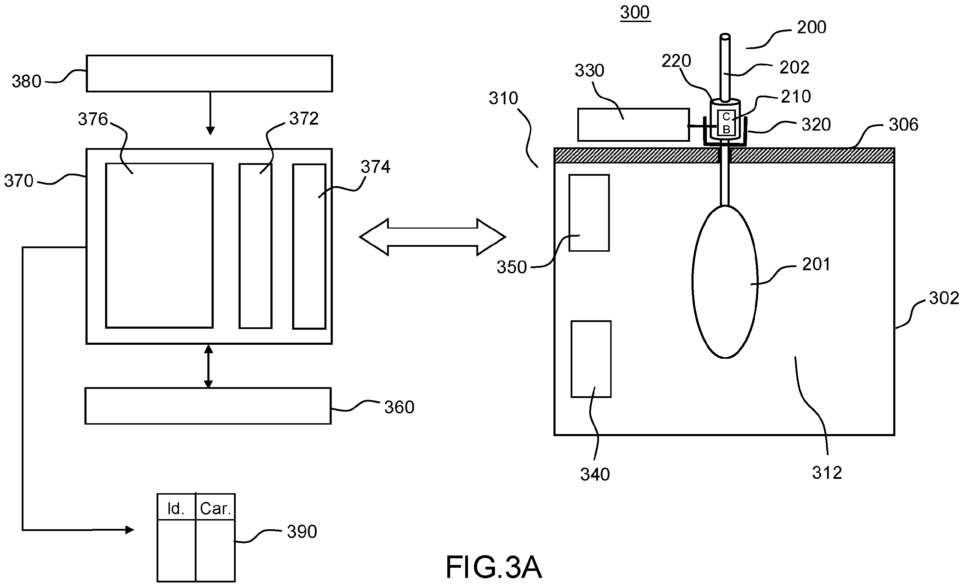

La FIG. 3A représente de façon schématique un exemple d'un système de désinfection 300 selon la présente description, dans une vue en coupe.

Le système de désinfection 300 est configuré pour mettre en oeuvre un cycle de désinfection d'au moins un instrument médical désigné par la référence 200 sur la FIG. 3A.

Bien que la présente description ne soit pas limitée à des procédés ou systèmes de désinfection pour la désinfection d'instruments médicaux critiques et semi-critiques, les procédés et systèmes décrits dans la présente description sont particulièrement adaptés à la DNI ou DNH de dispositifs et instruments médicaux réutilisables, y compris, par exemple, les sondes échographiques, endotrachéales et autres sondes endocavitaires comme les endoscopes ORL.

Un tel instrument médical comprend par exemple une partie active ou « tête » 201 et un câble de raccordement de la tête désigné par la référence 202, bien que des instruments médicaux sans câbles de raccordement puissent également être désinfectés au moyen des procédés et systèmes de désinfection décrits dans la présente description.

Le système de désinfection 300 comprend, dans l'exemple de la FIG. 3A, une ou plusieurs chambre(s) de désinfection 310 avec une enceinte 302 par exemple du type de celle illustrée sur la FIG. 1. La chambre de désinfection 310 comprend des parois latérales et un plafond 306. La chambre de désinfection 310 est configurée pour recevoir dans un volume intérieur 312 au moins une partie de l'instrument médical, par exemple la tête 201 et une partie du câble de raccordement 202.

Dans la présente description, les procédés et systèmes décrits dans la présente description utilisent un rayonnement ultraviolet (« UV ») pour effectuer rapidement une désinfection de haut niveau sans générer de températures trop élevées à la surface des instruments à traiter et à l'intérieur de ceux-ci.

Ainsi, dans l'exemple de la FIG. 3A, le système de désinfection est configuré pour la mise en oeuvre d'un cycle de désinfection par rayonnements UV.

Il comprend de façon connue une ou plusieurs sources de rayonnements UV référencées généralement par la référence 350 et un ou plusieurs capteurs desdits rayonnements UV référencées généralement par la référence 350. Le système de désinfection peut comprendre de manière connue d'autres capteurs, par exemple des capteurs de température.

Les sources de rayonnement qui peuvent être utilisées dans des procédés et systèmes selon la présente description sont par exemple des sources connues de l'état de l'art, et comprennent par exemple des sources de rayonnement pour l'émission de rayonnements UV-A, UV-B ou UV-C.

Des sources de rayonnements UV-C, également appelées « lampes » ou « tubes », sont disponibles dans le commerce et peuvent être obtenues sous différentes formes, tailles, énergie de sortie des rayonnements. Les tubes UV-C appropriés pour être utilisés comme sources de rayonnements UV-C comprennent par exemple des lampes à décharge à vapeur de mercure à basse pression. Cependant, toute source capable d'émettre des rayonnements UV-C dans la longueur d'onde UV-C sélectionnée à une énergie de sortie qui contribue à la désinfection d'un instrument médical cible 200 pourrait être utilisée dans les procédés et systèmes selon la présente description. Par exemple, en plus ou à la place d'un ou plusieurs tubes UV-C, un ou plusieurs lasers ou photodiodes, ou réseaux de sources, ou des combinaisons de types de sources conçues pour émettre de la lumière UV-C peuvent être utilisés pour délivrer des rayonnements dans la chambre de désinfection.

La ou les sources 350 peuvent comprendre également une ou plusieurs sources indirectes de rayonnement UV (par exemple des réflecteurs dédiés aux rayonnements).

Le ou les capteurs de rayonnements UV 340, par exemple une ou plusieurs photodiodes, peuvent être positionnés de manière fixe ou mobile dans le volume intérieur de la chambre de désinfection 310. Ils sont adaptés pour déterminer une quantité de rayonnements UV, par exemple une quantité de rayonnements mesurée en joules sur une surface sélectionnée ou, dans certains cas, une quantité de rayonnements spécifique à une surface, mesurée par exemple en joules / cm². Le ou les capteurs de rayonnements UV agencés dans le volume intérieur de la chambre de désinfection 310 peuvent être configurés pour avoir un filtre optique passe-bande ou tout autre filtre électromagnétique, de telle sorte que seul le rayonnement dans un spectre d'intérêt soit détecté.

Dans d'autres modes de réalisation, le ou les capteurs 340 peuvent comprendre un ou plusieurs composants conducteurs de lumière tels que des lentilles, des fibres optiques, des miroirs, des filtres et/ou d'autres éléments optiques utilisés pour collecter le rayonnement dans le volume intérieur de la chambre 310 vers un détecteur, tel qu'une photodiode.

La chambre de désinfection 310 est dimensionnée et configurée pour aider à réaliser la désinfection des instruments 200 placés dans celle-ci, dans une période de temps souhaitable, éventuellement sélectionnable, de sorte que les surfaces des instruments soient exposées à un niveau de rayonnement souhaité, appelé « dosage » dans la présente description. Un niveau d'exposition à un rayonnement, ou dosage, se rapporte à la fois à l'intensité du rayonnement et à la durée de l'exposition. Par exemple, l'instrument à désinfecter, la ou les sources de rayonnement UV et / ou le (s) capteur (s) de rayonnement UV sont positionnés (par exemple introduits, interposés, suspendus ou localisés) dans la chambre de désinfection à des positions fixes ou non, les positions pouvant être non stationnaires pendant un cycle de désinfection, afin de mieux exposer chacune des parties de surface de l'instrument à des niveaux de désinfection spécifiques de rayonnements UV, préalablement déterminés.

Le système de désinfection comprend en outre un dispositif de détection 330 configuré pour détecter, dans une zone de détection prédéterminée du système de désinfection, un marqueur 210 solidaire d'un instrument médical 200 devant subir un cycle de désinfection. Des exemples de marqueurs 210 et de dispositif de détection 330 seront décrits plus en détails par la suite.

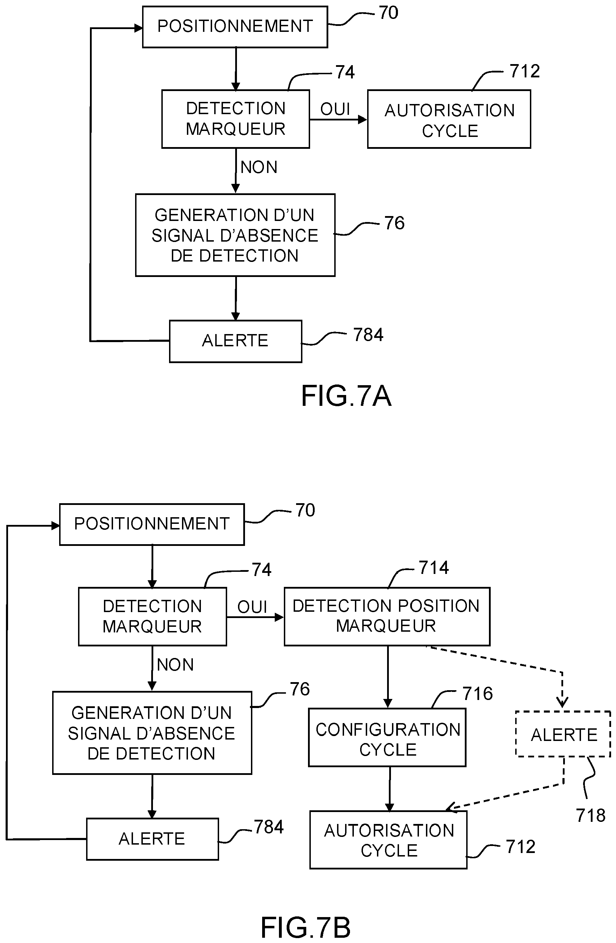

Comme cela sera décrit plus en détails au moyen des FIGS. 7A - 7D, un contrôleur 370 est configurée pour générer, avant et/ou pendant et/ou après un cycle de désinfection, un signal d'absence de détection en cas de non détection du marqueur 210 dans la zone de détection prédéterminée, et générer, le cas échéant, une ou plusieurs actions.

De façon générale, la chambre de désinfection 310 et/ou la/ou les sources de rayonnement 350 et/ou le ou les capteurs 340 de rayonnements UV et/ou le dispositif de détection 330 et/ou d'autres capteurs agencés dans la chambre de désinfection sont couplés en communication avec le contrôleur 370. Outre l'échange de données avec la chambre de désinfection, les sources de rayonnement, les capteurs et le dispositif de détection du marqueur, le contrôleur 370 peut également communiquer avec une ou plusieurs bases de données 360 et / ou une interface d'entrée 380 de données / informations relatives au contrôle de l'opération de désinfection, pour effectuer ses opérations.

Les informations relatives au contrôle de l'opération de désinfection peuvent comprendre des informations de caractérisation du cycle de décontamination, par exemple : des informations d'identification de la sonde et/ou de l'enceinte, chaque enceinte étant alors affectée à un numéro d'identification spécifique stocké dans celle-ci, des informations d'horodatage du cycle permettant par exemple d'acquérir la date du cycle, le numéro journalier du cycle, l'heure de début et l'heure de fin du cycle, à partir d'un circuit formant horloge, etc. Les informations de caractérisation du cycle de décontamination peuvent également comporter des informations relatives à la dose d'UV émise lors d'un cycle si l'enceinte est une enceinte de désinfection munie de moyens de génération de rayonnement UV de désinfection. Par exemple, afin d'assurer la traçabilité du cycle, les informations relatives au contrôle de l'opération de désinfection peuvent comprendre des informations d'identification de la sonde et/ou de l'enceinte, des informations d'identification du patient qui bénéficiera de la désinfection, ainsi que des informations d'identification de l'opérateur qui a réalisé la désinfection.

Le contrôleur peut ainsi contrôler l'exécution d'un cycle de désinfection, déterminer un dosage minimal à appliquer dans la chambre de désinfection, pour une désinfection optimale de l'instrument, et contrôler le positionnement de l'instrument.

Le contrôleur 370 peut comprendre de façon connue un processeur 372, une ou plusieurs unités de stockage ou mémoires 376 et une interface 374 configurée pour communiquer avec la chambre de désinfection 310, la/ou les sources de rayonnement 350, le ou les capteurs 340, le dispositif de détection 330. L'interface 374 comprend par exemple une convertisseur CAN pour convertir des données analogiques, telles que les données détectées par les capteurs, en données numériques pouvant être traitées par le processeur 372. Un processeur 372 tel que décrit dans la présente description comprend tout dispositif, par exemple une unité centrale de traitement (CPU), un microprocesseur, un microcontrôleur (MCU), un processeur de signal numérique (DSP), un circuit intégré spécifique à une application (ASIC), ou partie de celui-ci, qui commande au moins une opération ; il peut être implémenté sous forme de matériel, de microprogramme ou de logiciel, ou une combinaison. La fonctionnalité associée à un processeur particulier peut être centralisée ou distribuée, que ce soit localement ou à distance. Un processeur peut faire référence de manière interchangeable à tout type de circuit de commande électronique configuré pour exécuter des instructions logicielles programmées. La ou les mémoires 376 peuvent comprendre de façon connue toute combinaison de supports lisibles par ordinateur volatils et non volatils pour la lecture et l'écriture. Les supports volatiles lisibles par ordinateur comprennent, par exemple, la mémoire vive (RAM). Un support lisible par ordinateur non volatile comprend, par exemple, une ou plusieurs mémoires ROM, des supports magnétiques tels qu'un disque dur, un disque optique, un dispositif de mémoire flash, un CD-ROM, etc. Tout ou partie du contenu stocké d'une mémoire peut inclure des instructions logicielles exécutables par un dispositif de traitement afin de réaliser un ou plusieurs actes particuliers.

Parmi les instructions stockées, ces instructions, de façon indicative mais non limitative, peuvent comprendre une pluralité de sondes acceptées et une pluralité de doses à appliquer.

Le marqueur 210 est solidaire de l'instrument médical, c'est-à-dire qu'il ne peut être retiré et/ou déplacé sans détérioration du marqueur lui-même et/ou de l'instrument médical sur lequel il est apposé. Le marqueur peut être un élément externe apposé de façon solidaire à l'instrument, par exemple par collage ou intégration au sein de l'instrument, par exemple une étiquette ou une puce électronique. Le marqueur peut également être un élément de l'instrument lui-même. Avec un marqueur solidaire de l'instrument, il est possible de contrôler de façon certaine la position de l'instrument médical dans le système de désinfection.

Le marqueur 210 peut être porté par la partie active 201 de l'instrument ou encore par le câble de raccordement 202 de l'instrument, comme cela est illustré sur la FIG. 3A.

Le dispositif de détection 330 peut-être placé à l'extérieur du volume intérieur de la chambre de désinfection, comme illustré sur la FIG. 3A, ou dans le volume intérieur 312, par exemple au niveau des parois latérales de celle-ci, par exemple si le marquer est positionné sur la partie active de l'instrument ou à proximité de celle-ci.

De façon générale, le marqueur peut comprendre tout élément de nature à pouvoir être détecté de façon spécifique par le dispositif de détection.

Par exemple, le marqueur peut être une puce électronique de type puce RFID (abréviation de l'expression anglo-saxonne « *radio frequency identification* ») et le dispositif de détection peut comprendre dans ce cas des moyens d'acquisition radiofréquence configurés pour la détection de la puce électronique.

Selon d'autres exemples, le dispositif de détection 330 est un dispositif de détection optique, sensible à un rayonnement visible ou infrarouge.

Il peut comprendre par exemple un ou plusieurs détecteur(s) ponctuel(s), par exemple une ou plusieurs photodiode(s) et/ou un ou plusieurs détecteur(s) matriciel(s), par exemple une caméra linéaire ou une caméra bidimensionnelle, par exemple une caméra CCD ou CMOS. Le dispositif de détection 330 peut comprendre également lecteur code-barres à une ou deux dimensions pour l'identification de l'instrument médical, comme cela sera décrit plus en détails par la suite.

De façon générale, le dispositif de détection est adapté en fonction du marqueur.

Le marqueur peut comprendre selon un ou plusieurs exemples et de façon non limitative un élément choisi parmi le groupe comprenant : un élément coloré, un élément fluorescent, une sérigraphie, un élément réfléchissant, un élément diffractif, un code-barres, ou une combinaison de ces éléments.

Ainsi, une ou plusieurs caméra(s) sont par exemple adaptée(s) à un marqueur formé d'un élément structurel de l'instrument médical lui-même et identifié comme marqueur pour l'instrument. Un détecteur ponctuel, par exemple de type photodiode, est adapté à un marqueur ponctuel tel qu'un un point absorbant ou réfléchissant une onde dans une proportion différente du reste des éléments de la sonde, tandis qu'un lecteur code-barres est adapté à un marqueur de type code-barres. Un autre exemple non limitatif de détection optique peut comprendre un laser.

Selon un ou plusieurs exemples de réalisation, le dispositif de détection comprend en outre une ou plusieurs source(s) d'émission d'un rayonnement lumineux configurée(s) pour éclairer ladite zone de détection. La source d'émission permet la détection même lorsque, pendant un cycle de fonctionnement, la zone de détection se trouve dans une région non éclairée du système de désinfection, par exemple un espace fermé à la lumière du jour.

Selon un ou plusieurs exemples de réalisation, le dispositif de détection est configuré en outre pour détecter, dans ladite zone de détection, une position du marqueur par rapport à une position de référence. Le contrôleur 370 peut dans ce cas être configuré pour générer, à partir de ladite position du marqueur dans la zone de détection, la reconfiguration d'un cycle de désinfection pour tenir compte de ladite position.

Selon un exemple de la présente description, le marqueur 210 solidaire de l'instrument médical comprend un code d'identification de l'instrument. Ce code d'identification peut être mémorisé par exemple sous forme d'un code barre à une ou deux dimensions ou dans une étiquette électronique radiofréquence.

Le dispositif de détection 330 et le contrôleur 370 sont alors configurés pour l'acquisition des informations d'identification portées par le code d'identification de l'instrument, en addition de la détection de la présence ou non de l'instrument dans la zone de détection. Par exemple le dispositif de détection 330 comprend un lecteur code-barres dans le cas d'un marqueur de type code-barre ou des moyens radiofréquence d'acquisition dans le cas d'une étiquette électronique radiofréquence. Les informations d'identification de l'instrument médical peuvent être acquises lors de la mise en place de l'instrument médical dans la chambre de désinfection et/ou lors de son retrait de la chambre de désinfection et/ou au début et/ou à la fin d'un cycle de désinfection.

Les informations d'identification du ou de chaque instrument, couplées avec des informations détection de l'instrument et de caractérisation du cycle de désinfection, permettent de générer des informations de traçabilité de la désinfection du ou de chaque instrument, référencées 390 sur la FIG. 3A. Ces informations de traçabilité permettent par exemple de mettre en relation chaque instrument avec les conditions dans lesquelles s'est déroulé le cycle de désinfection correspondant et peuvent être optionnellement associées à des informations relatives à l'identité du patient qui a bénéficié de la désinfection, et/ou à des informations relatives à l'opérateur qui a réalisé la désinfection. Ces informations de traçabilité peuvent être stockées, visualisées sur un afficheur ou transmises à un ou plusieurs opérateur(s) à distance. Elles permettent de garantir le passage de l'instrument dans la chambre de désinfection et de vérifier les informations de caractérisation du cycle de désinfection subi par l'instrument, c'est-à- dire notamment le moment où cette désinfection a eu lieu, la chambre dans laquelle s'est déroulé le cycle de désinfection, la validation d'un bon positionnement de l'instrument dans la chambre de désinfection, la dose d'UV reçue par l'instrument, la température lors du cycle, le fait que l'instrument était bien nettoyé avant le cycle de désinfection.

Selon un ou plusieurs exemples de réalisation, le marqueur peut être fourni sous la forme d'un kit de détection pour un instrument médical destiné à être désinfecté dans un système de désinfection selon la présente description. Le kit de détection comprend ainsi un marqueur configuré pour être apposé solidairement sur ledit instrument médical en vue de sa détection par le dispositif de détection.

Le kit de détection peut être utilisé par un opérateur en amont d'un cycle de désinfection pour « marquer » un instrument médical en vue de sa détection dans le système de désinfection avant et/ou pendant et/ou après un cycle de désinfection.

Le marqueur d'un kit de détection peut comprendre avantageusement un code d'identification de l'appareil médical.

En pratique, un opérateur pourra apposer le marquer de façon solidaire sur l'instrument médical, à une position prédéterminée de celui-ci. Par exemple, un installateur du système de désinfection pourra appliquer un étiquette type code barre à un certain endroit du câble de l'instrument, recouvrir cette étiquette par un élément enserrant le câble et rendant l'étiquette inamovible dans des conditions d'utilisation normale. Cet élément peut être configuré de façon à permettre une accroche systématiquement répétable. Selon un ou plusieurs exemples de réalisation, l'élément peut former un couple mâle-femelle avec une des pièces de l'enceinte, et peut être muni d'une partie transparente, par exemple un vitre, qui laisse passer les rayons du lecteur du code d'identification, par exemple, les rayons du lecteur de code barre.

Selon un ou plusieurs exemples de réalisation, une puce RFID peut être appliquée à un endroit prédéterminé du câble et couplée à un lecteur RFID à champ de détection droit et restreint. Cette forme de réalisation peut remplir la fonction de détecter, dans la zone de détection, une position du marqueur par rapport à une position de référence.

Selon d'autres exemples de réalisation, on peut concevoir également des instruments médicaux marqués, configurés pour être désinfectés dans un système de désinfection selon la présente description. Chaque instrument médical marqué comprend un marqueur apposé solidairement sur une région dudit instrument médical en vue de sa détection par le dispositif de détection. Le marqueur est ainsi formé d'un élément externe à l'instrument et configuré pour la détection de l'instrument médical lorsqu'il est positionné dans le système de désinfection, en vue d'un cycle de désinfection. Selon un ou plusieurs exemples de réalisation, le marqueur comprend un code d'identification de l'instrument médical. Un tel instrument médical marqué pourra être fourni par exemple avec le système de désinfection.

Selon d'autres exemples de réalisation, un marqueur peut être intégré dans des instruments médicaux déjà fabriqués. Par exemple, selon un ou plusieurs exemples de réalisation, une sonde peut intégrer dans un câble un identifiant dans une forme qui peut s'emboiter dans une pièce d'intérêt de l'enceinte de la chambre de désinfection positionnant l'identifiant dans le champ de détection du lecteur.

Dans l'exemple de la FIG. 3A, le marqueur 210 comprend par exemple une étiquette avec un code d'identification ; il est porté par le câble de raccordement et est détecté, en opération, par le dispositif de détection 330, par exemple un lecteur code-barre agencé à l'extérieure du volume intérieur de la chambre de désinfection. Le lecteur de code barre présente un champ donné qui définit une zone de détection.

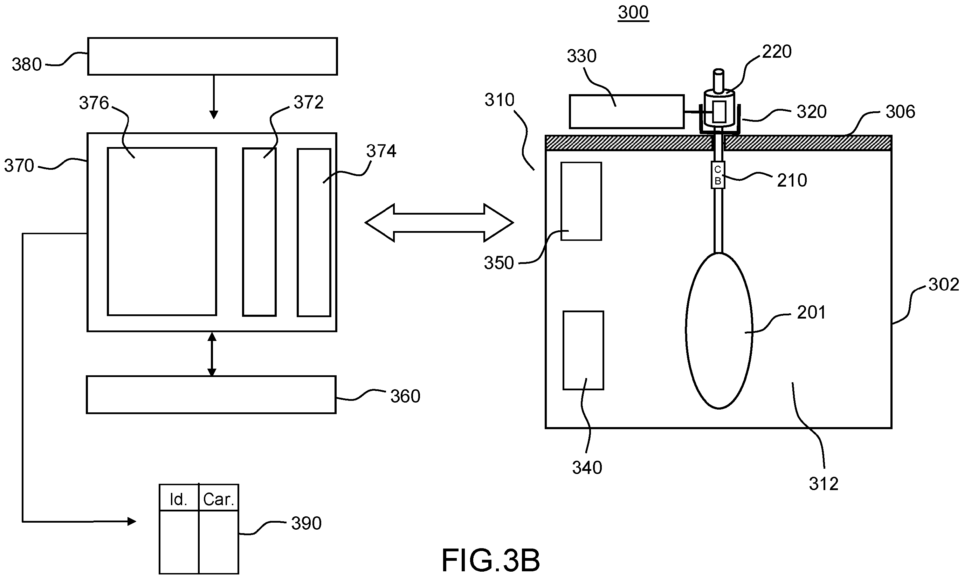

Comme illustré sur la FIG. 3B, si pour une raison ou pour une autre, l'instrument médical n'est pas dans la position prédéfinie pour la désinfection, le détecteur optique 330 ne détecte pas dans la zone de détection le marqueur, ce qui entraîne la génération d'un signal d'absence de détection par le contrôleur 370.

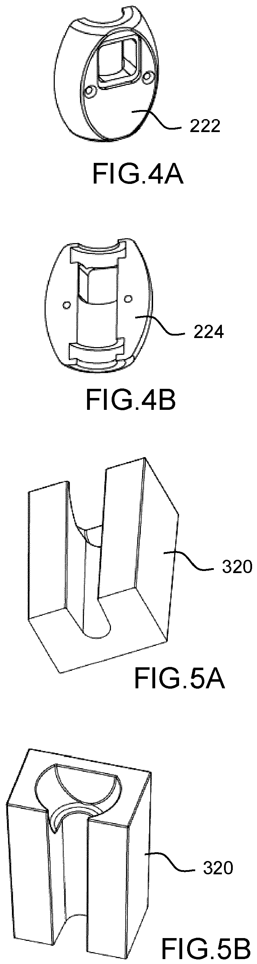

Dans l'exemple de la FIG. 3A, la chambre de désinfection 310 est configurée pour permettre la suspension de l'instrument médical dans le volume intérieur 312, par exemple au moyen d'une bague 220 coopérant avec un organe de suspension 320, comme cela est décrit plus en détails au moyen des FIGS 4A, 4B et 5A, 5B.

L'organe de suspension 320 illustré selon deux vues sur les FIGS 5A et 5B comprend un corps dans lequel une cavité de forme complémentaire à au moins une partie de l'instrument médical est définie.

L'organe de suspension coopère avec la bague 220 configurée pour être fixée par exemple sur le câble 202 de l'instrument médical 200 de telle sorte à laisser apparent le marqueur 210. Par exemple, dans le cas d'une détection optique du marqueur 210, la bague 220 comprend une fenêtre 221 destinée à être placé au niveau du marqueur. En cas de glissement non voulue de la bague 220 sur le câble, comme illustré par exemple sur la FIG. 3B, le marqueur 210 qui est solidaire de l'instrument médical 200 ne sera plus détecté par le dispositif de détection 330.

Bien entendu, d'autres moyens peuvent être envisagés pour la mise en place de l'instrument médical dans la chambre 310.

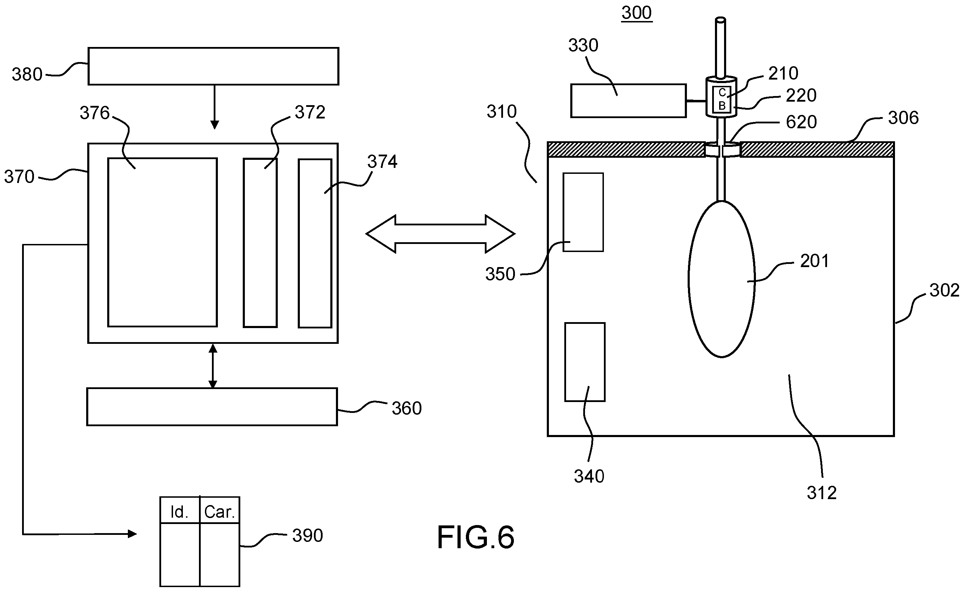

Ainsi, dans l'exemple de réalisation d'un système de désinfection de la FIG. 6, un organe de suspension 620 en forme d'anneau logé dans une ouverture du plafond 306 est illustré.

Les FIGS 7A à 7D représentent des schémas illustrant des exemples de mise en oeuvre de procédés de détection d'un instrument médical dans un système de désinfection selon la présente description.

Un procédé de détection d'un instrument médical selon la présente description comprend de façon générale la détection 74 dans une zone de détection prédéterminée du système de désinfection, au moyen d'un dispositif de détection, d'un marqueur solidaire dudit instrument médical ; et en cas de non détection dudit marqueur dans ladite zone de détection prédéterminée du système de désinfection, la génération 76 d'un signal d'absence de détection.

La détection du marqueur peut se faire avant et/ou pendant et/ou à la fin d'un cycle de désinfection.

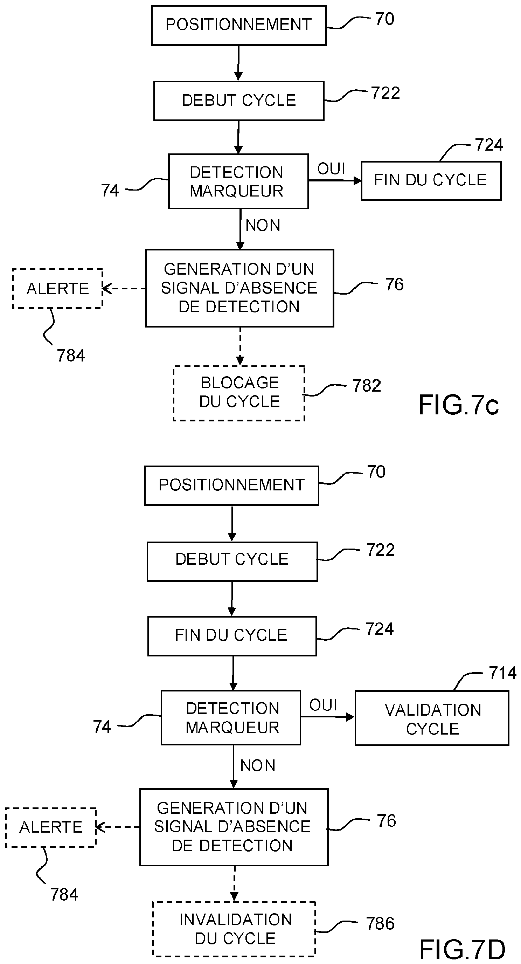

Les FIGS. 7A et 7B illustrent des exemples de procédés dans lesquels la détection du marqueur est fait avant le lancement d'un cycle de détection. La FIG. 7C illustre un exemple de procédé dans lequel la détection du marqueur est fait pendant un cycle de détection et la FIG. 7D illustre un exemple de procédé dans lequel la détection du marqueur est fait après un cycle de détection. Bien entendu, ces exemples ne sont pas limitatifs et peuvent être combinés.

Plus précisément, l'exemple de procédé illustré sur la FIG. 7A comprend, après le positionnement 70 de l'instrument médical, la détection 74 dans la zone de détection du marqueur solidaire de l'instrument médical. Si le marqueur est détecté, le cycle peut être autorisé (712). En cas de non détection du marqueur dans la zone de détection, le procédé comprend la génération 76 d'un signal d'absence de détection. Il s'ensuit par exemple la génération d'une alerte (784) à destination d'un utilisateur qui pourra, le cas échéant, vérifier et corriger la position de l'instrument médical dans le système de désinfection.

L'exemple de procédé illustré sur la FIG. 7B représente une variante de celui illustré sur la FIG. 7A dans lequel en cas de détection du marqueur dans la zone de détection, il est procédé en outre à la détection 714, dans ladite zone de détection, d'une position du marqueur par rapport à une position de référence. Il s'ensuit, à partir de ladite position du marqueur dans la zone de détection, une ou plusieurs actions comprenant par exemple : l'autorisation de lancement du cycle (712), l'émission 718 d'une alerte de défaut de positionnement à destination d'un ou plusieurs utilisateur(s) et/ou la reconfiguration 716 d'un cycle de désinfection pour tenir compte de ladite position.

L'exemple de procédé illustré sur la FIG. 7C comprend, après le positionnement 70 de l'instrument médical et le lancement d'un cycle (722), la détection 74 dans la zone de détection du marqueur solidaire de l'instrument médical. Si le marqueur est détecté, le cycle peut se poursuivre normalement (724). En cas de non détection du marqueur dans la zone de détection, le procédé comprend la génération 76 d'un signal d'absence de détection. Il s'ensuit par exemple la génération d'une alerte (784) à destination d'un utilisateur ou le blocage du cycle (782).

L'exemple de procédé illustré sur la FIG. 7D comprend, après le positionnement 70 de l'instrument médical et la réalisation complète d'un cycle de désinfection (722, 724), la détection 74 dans la zone de détection du marqueur solidaire de l'instrument médical. Si le marqueur est détecté, le cycle est validé (714). En cas de non détection du marqueur dans la zone de détection, le procédé comprend la génération 76 d'un signal d'absence de détection. Il s'ensuit par exemple la génération d'une alerte (784) à destination d'un utilisateur ou une invalidation du cycle (786).

Dans les exemples de procédés décrits ci-dessus, la détection du marqueur peut se faire selon l'un quelconque des exemples décrits précédemment. Par exemple, il peut s'agir d'une détection optique d'un marqueur pouvant prendre différentes formes, dont par exemple un élément externe (par exemple une étiquette) apposé de façon solidaire à l'instrument, par exemple par collage ou intégration au sein de l'instrument, ou un élément de l'instrument lui-même.

Dans chacun des exemples de procédés décrits au moyen des FIGS 7A - 7D, lorsque le marqueur comprend un code d'identification de l'instrument médical, le procédé peut comprendre en outre la lecture du code d'identification au moyen du dispositif de détection (étape non représentée sur les figures).

Les procédés décrits peuvent comprendre en outre une étape préalable de marquage de l'instrument médical, par apposition dudit marqueur de façon solidaire sur ledit instrument médical, lorsque le marqueur est un élément externe à l'instrument (étape non représentée sur les figures).

## Revendications

1. Kit de désinfection pour un instrument médical (200) comprenant un système de désinfection (300) et un kit de détection pour l'instrument médical (200), le système de désinfection (300) étant configuré pour mettre en oeuvre un cycle de désinfection d'un instrument médical (200) par rayonnements UV, le système de désinfection (300) comprenant :
- une chambre de désinfection (310) configurée pour recevoir au moins une partie de l'instrument médical (200);
- un organe de suspension (320) permettant de suspendre ladite au moins une partie de l'instrument médical (200) dans ladite chambre de désinfection (310) ;
- un dispositif de détection (330) configuré pour détecter, dans une zone de détection prédéterminée du système de désinfection (300), un marqueur (210) solidaire dudit instrument médical (200) ;
- un contrôleur (370) configuré pour générer un signal d'absence de détection en cas de non détection dudit marqueur (210) dans ladite zone de détection prédéterminée,
- le kit de détection étant destiné à être désinfecté dans le système de désinfection (300), ledit instrument médical (200) comprenant une tête (201) et un câble de raccordement (202) de la tête (201),
- le kit de détection comprenant :
- un marqueur (210) configuré pour être apposé solidairement sur une partie du câble de raccordement (202) voisine de la tête (201) en vue de sa détection par ledit dispositif de détection (330) ; et
- une bague de protection (220) dudit marqueur (210), ladite bague de protection (220) coopérant en outre avec ledit organe de suspension (320) pour la suspension du câble de raccordement (202).

2. Kit de désinfection selon la revendication 1, dans lequel le dispositif de détection (330) comprend un dispositif de détection optique.

3. Kit de désinfection selon la revendication 2, dans lequel le dispositif de détection (330) comprend en outre une source d'émission d'une radiation lumineuse configurée pour éclairer ladite zone de détection prédéterminée.

4. Kit de désinfection selon la revendication 2 ou la revendication 3, dans lequel le dispositif de détection (330) comprend un lecteur de code-barres.

5. Kit de désinfection selon la revendication 1, dans lequel le dispositif de détection (330) comprend des moyens d'acquisition radiofréquence.

6. Kit de désinfection selon l'une quelconque des revendications 1 à 5, comprenant au moins une source (350) de rayonnements UV.

7. Kit de désinfection selon la revendication 1 à 6, dans lequel le marqueur (210) comprend un code d'identification dudit instrument médical (200).

8. Procédé de détection d'un instrument médical (200) dans un kit de désinfection selon l'une quelconque des revendications 1 à 7, le procédé comprenant les étapes suivantes :
- la détection (74) dans ladite zone de détection prédéterminée du système de désinfection (300), au moyen dudit dispositif de détection (330), dudit marqueur (210) solidaire dudit instrument médical (200); et
- en cas de non détection dudit marqueur (210) dans ladite zone de détection prédéterminée du système de désinfection (300), la génération (76) d'un signal d'absence de détection.

9. Procédé de détection selon la revendication 8, dans lequel la génération du signal d'absence de détection entraine l'émission d'une alerte à destination d'un ou plusieurs utilisateur(s) et/ou un arrêt d'un cycle de désinfection en cours et/ou une interdiction de lancement d'un cycle.

10. Procédé de détection selon la revendication 8 ou la revendication 9, dans lequel le marqueur (210) comprend un code d'identification de l'instrument médical (200) et le procédé comprend en outre :
- la lecture du code d'identification au moyen du dispositif de détection (330).

11. Procédé de détection selon l'une quelconque des revendications 8 à 10, comprenant une étape préalable de marquage de l'instrument médical (200), par apposition du marqueur (210) de façon solidaire sur ledit instrument médical (210).

12. Procédé de détection selon l'une quelconque des revendications 8 à 11, dans lequel la détection est une détection optique ou une détection radio.

13. Procédé de détection selon l'une quelconque des revendications 8 à 12, comprenant en outre la détection (714), dans ladite zone de détection, d'une position du marqueur (210) par rapport à une position de référence.

14. Procédé de détection selon la revendication 13, comprenant en outre, à partir de ladite position du marqueur (210) dans la zone de détection, une ou plusieurs actions comprenant : émission d'une alerte de défaut de positionnement à destination d'un ou plusieurs utilisateur(s) et/ou reconfiguration d'un cycle de désinfection pour tenir compte de ladite position et/ou arrêt du cycle de désinfection en cours et/ou interdiction de lancement d'un cycle de désinfection.

## Patentansprüche

1. Desinfektionsset für ein medizinisches Instrument (200), das ein Desinfektionssystem (300) und ein Detektionsset für das medizinische Instrument (200) umfasst, wobei das Desinfektionssystem (300) konfiguriert ist, um einen Desinfektionszyklus eines medizinischen Instruments (200) durch LIV-Strahlung zu implementieren, wobei das Desinfektionssystem (300) Folgendes umfasst:
- eine Desinfektionskammer (310), die so konfiguriert ist, dass sie mindestens einen Teil des medizinischen Instruments (200) aufnimmt;
- ein Aufhängeelement (320) zum Aufhängen des mindestens einen Teils des medizinischen Instruments (200) in der Desinfektionskammer (310);
- eine Erfassungsvorrichtung (330), die so konfiguriert ist, dass sie in einem vorbestimmten Erfassungsbereich des Desinfektionssystems (300) einen Markierer (210) erfasst, der fest mit dem medizinischen Instrument (200) verbunden ist;
- eine Steuereinheit (370), die so konfiguriert ist, dass sie ein Nicht-Erfassungssignal erzeugt, wenn der Markierer (210) in dem vorbestimmten Erfassungsbereich nicht erfasst wird,
- wobei das Detektionsset dazu bestimmt ist, in dem Desinfektionssystem (300) desinfiziert zu werden, wobei das medizinische Instrument (200) einen Kopf (201) und ein Verbindungskabel (202) des Kopfes (201) umfasst,
- das Detektionsset bestehend aus:
- einem Markierer (210), der so konfiguriert ist, dass er fest an einem Teil des Verbindungskabels (202), der dem Kopf (201) benachbart ist, angebracht wird, um von der Erfassungsvorrichtung (330) erfasst zu werden; und
- einem Schutzring (220) des Markierers (210), wobei der Schutzring (220) außerdem mit dem Aufhängeelement (320) zum Aufhängen des Verbindungskabels (202) zusammenwirkt.

2. Desinfektionsset nach Anspruch 1, wobei die Erfassungsvorrichtung (330) eine optische Erfassungsvorrichtung umfasst.

3. Desinfektionsset nach Anspruch 2, wobei die Erfassungsvorrichtung (330) außerdem eine Quelle zur Emission von Lichtstrahlung umfasst, die so konfiguriert ist, dass sie den vorbestimmten Erfassungsbereich beleuchtet.

4. Desinfektionsset nach Anspruch 2 oder Anspruch 3, wobei die Erfassungsvorrichtung (330) einen Strichcodeleser umfasst.

5. Desinfektionsset nach Anspruch 1, wobei die Erfassungsvorrichtung (330) ein Radiofrequenzerfassungsmittel umfasst.

6. Desinfektionsset nach einem der Ansprüche 1 bis 5, das mindestens eine Quelle (350) für UV-Strahlung umfasst.

7. Desinfektionsset nach Anspruch 1 bis 6, wobei der Markierer (210) einen Identifikationscode des medizinischen Instruments (200) enthält.

8. Erfassungsverfahren eines medizinischen Instruments (200) in einem Desinfektionsset nach einem der Ansprüche 1 bis 7, wobei das Verfahren die folgenden Schritte umfasst:
- Erfassen (74) in dem vorbestimmten Erfassungsbereich des Desinfektionssystems (300) mittels der Erfassungsvorrichtung (330), des Markierers (210), der mit dem medizinischen Instrument (200) verbunden ist; und
- bei Nicht-Erfassung des Markierers (210) in dem vorbestimmten Erfassungsbereich des Desinfektionssystems (300), Erzeugen (76) eines Nicht-Erfassungssignals.

9. Erfassungsverfahren nach Anspruch 8, wobei das Erzeugen des Nicht-Erfassungssignals dazu führt, dass eine Warnung an einen oder mehrere Benutzer ausgegeben wird und/oder ein laufender Desinfektionszyklus angehalten wird und/oder der Start eines Zyklus verhindert wird.

10. Erfassungsverfahren nach Anspruch 8 oder Anspruch 9, wobei der Markierer (210) einen Identifikationscode des medizinischen Instruments (200) umfasst und das Verfahren ferner umfasst:
- das Lesen des Identifikationscodes mithilfe der Erfassungsvorrichtung (330).

11. Erfassungsverfahren nach einem der Ansprüche 8 bis 10, umfassend einen vorherigen Schritt des Markierens des medizinischen Instruments (200), indem der Markierer (210) fest an dem medizinischen Instrument (210) angebracht wird.

12. Erfassungsverfahren nach einem der Ansprüche 8 bis 11, wobei die Erfassung eine optische Erfassung oder eine Funkdetektion ist.

13. Erfassungsverfahren nach einem der Ansprüche 8 bis 12, das außerdem das Erfassen (714) einer Position des Markierers (210) in Bezug auf eine Referenzposition in dem Erfassungsbereich umfasst.

14. Erfassungsverfahren nach Anspruch 13, das außerdem ausgehend von der Position des Markierers (210) in dem Erfassungsbereich eine oder mehrere Aktionen umfasst, die Folgendes umfassen: Ausgabe einer Fehlpositionswarnung an einen oder mehrere Benutzer und/oder Neukonfiguration eines Desinfektionszyklus zur Berücksichtigung der genannten Position und/oder Anhalten des laufenden Desinfektionszyklus und/oder Verbot des Starts eines Desinfektionszyklus.

## Claims

1. A disinfection kit for a medical instrument (200) comprising a disinfection system (300) and a detection kit for the medical instrument (200), the disinfection system (300) being configured to implement a disinfection cycle of a medical instrument (200) by UV radiation, the disinfection system (300) comprising:
- a disinfection chamber (310) configured to receive at least one portion of the medical instrument (200);
- a suspension means (320) for suspending said at least one portion of the medical instrument (200) in said disinfection chamber (310);
- a detection device (330) configured to detect a marker (210) integral with said medical instrument (200), in a predetermined detection area of the disinfection system (300);
- a controller (370) configured to generate a non-detection signal in case of non-detection of said marker (210) in said predetermined detection area,
- the detection kit (200) being intended for disinfection in the disinfection system (300), said medical instrument (200) comprising a head (201) and a connection cable (202) of the head (201),
- the detection kit comprising:
- a marker (210) configured to be integrally affixed to a portion of the connection cable (202) adjacent the head (201) for detection by said detection device (330); and
- a protective ring (220) of said marker (210), said protective ring (220) further interacting with said suspension means (320) for suspending the connection cable (202).

2. The disinfection kit according to claim 1, wherein the detection device (330) comprises an optical detection device.

3. The disinfection kit according to claim 2, wherein the detection device (330) further comprises a source for emitting light radiation configured to illuminate said predetermined detection area.

4. The disinfection kit according to claim 2 or claim 3, wherein the detection device (330) comprises a barcode reader.

5. The disinfection kit according to claim 1, wherein the detection device (330) comprises radio frequency acquisition means.

6. The disinfection kit according to any one of claims 1 to 5, comprising at least one source (350) of UV radiation.

7. The disinfection kit according to any one of claims 1 to 6, wherein the marker (210) comprises an identification code of said medical instrument (200).

8. A method for detecting a medical instrument (200) in a disinfection kit according to any of claims 1 to 7, the method comprising the following steps:
- detecting (74) said marker (210) integral with said medical instrument (200) in said predetermined detection area of the disinfection system (300), by means of said detection device (330); and
- in case of non-detection of said marker (210) in said predetermined detection area of the disinfection system (300), generating (76) a non-detection signal.

9. The detection method according to claim 8, wherein generation of the non-detection signal results in issuing an alert to one or more users and/or stopping a disinfection cycle in progress and/or prohibiting the launch of a cycle.

10. The detection method according to claim 8 or claim 9, wherein the marker (210) comprises an identification code of the medical instrument (200) and the method further comprises:
- reading the identification code using the detection device (330).

11. The detection method according to any one of claims 8 to 10, comprising a prior step of marking the medical instrument (200), by affixing the marker (210) integrally to said medical instrument (210).

12. The detection method according to any one of claims 8 to 11, wherein the detection is optical detection or radio detection.

13. The detection method according to any one of claims 8 to 12, further comprising detecting (714), in said detection area, a position of the marker (210) relative to a reference position.

14. The detection method according to claim 13, further comprising one or more actions, based on said position of the marker (210) in the detection area, comprising: issuing a positioning error alert to one or more user(s) and/or reconfiguring a disinfection cycle to take account of said position and/or stopping the disinfection cycle in progress and/or prohibiting the launch of a disinfection cycle.
